(19) Europäisches Patentamt
European Patent Office
Office européen des brevets

(11) Veröffentlichungsnummer: **0 090 977**
**B2**

(12) # NEUE EUROPÄISCHE PATENTSCHRIFT

(45) Veröffentlichungstag der neuen Patentschrift: **14.09.88**

(51) Int. Cl.[4]: **C 07 C 69/96,** C 07 C 68/00

(21) Anmeldenummer: **83102668.7**

(22) Anmeldetag: **18.03.83**

(54) **Verfahren zur Herstellung von Kohlensäureestern.**

(30) Priorität: **03.04.82 DE 3212535**

(43) Veröffentlichungstag der Anmeldung:
**12.10.83 Patentblatt 83/41**

(45) Bekanntmachung des Hinweises auf die Patenterteilung:
**24.07.85 Patentblatt 85/30**

(45) Bekanntmachung des Hinweises auf die Entscheidung über den Einspruch:
**14.09.88 Patentblatt 88/37**

(84) Benannte Vertragsstaaten:
**DE FR GB IT NL**

(56) Entgegenhaltungen:
**EP - A - 0 038 984**
**US - A - 3 114 762**
**US - A - 4 361 519**

**J.Org. Chem. 39(5),S.701-704(1974)**
**Ind.Eng.Chem.Prod.Res.Dev.1980,19, 396-403**
**Ullmanns Encyklopädie der technischen Chemie, 4.Auflage, Bd.17 (1979). 500-501**

(73) Patentinhaber: **Röhm GmbH, Kirschenallee Postfach 4242, D-6100 Darmstadt 1 (DE)**

(72) Erfinder: **Baumgartner, Ehrenfried, Dr. Dipl.-Chem., Reutterallee 44, D-6100 Darmstadt (DE)**
Erfinder: **Schröder, Günter, Dr. Dipl.-Chem., Leipziger Strasse 7, D-6105 Ober-Ramstadt (DE)**
Erfinder: **Besecke, Siegmund, Dr. Dipl.-Chem., Auf dem Kreuzberg 6, D-6104 Seeheim-Jugenheim (DE)**

**EP 0 090 977 B2**

**Beschreibung**

Gebiet der Erfindung

Die Erfindung betrifft die Herstellung von Kohlensäureestern. Kohlensäureester, insbesondere die symmetrischen Diester, werden technisch als Zwischenprodukte und Lösungsmittel verwendet.

Stand der Technik

Bekannte Syntheseverfahren zur Herstellung von Kohlensäureestern gehen von Phosgen oder Äthylenoxid aus. Zu diesen Verfahren trat die Umsetzung von Alkoholen mit Kohlenmonoxid und Sauerstoff oder anderen Oxidationsmitteln. Für die unter Verbrauch von Sauerstoff verlaufende einstufige Reaktion (siehe Gleichung) sind bestimmte Katalysatoren vorgeschlagen worden:

$$2ROH + CO + \frac{1}{2} O_2 \xrightarrow{\text{Kat.}} (RO)_2CO + H_2O$$

In der DE-A 2 110 194 wurden als geeignete Katalysatoren Metallkomplexe der Gruppen IB, IIB und VIII des Periodensystems genannt, insbesondere die Metalle Cu, Ag, Au, Zn, Cd, Hg, Fe, Co, Ni, die bei Redoxreaktionen in zwei verschiedenen Valenzzuständen existieren können. Nach den beispielhaften Ausführungen findet bei 1 bar Reaktionsdruck ausschliesslich stöchiometrische Umsetzung mit dem verwendeten $Cu_2Cl_2$ statt. Ein Beispiel einer Umsetzung unter erhöhtem Druck in wasserfreiem Pyridin wird angegeben, aus dem jedoch die katalytische Wirkung nicht klar beurteilt werden kann.

In der DE-A 2 743 690 wird darauf hingewiesen, dass das Verfahren der vorgenannten DE-A bei grosstechnischer Durchführung schwerwiegende Nachteile aufweist, wie z.B. Empfindlichkeit gegenüber Wasser und Kohlendioxid, die als Nebenprodukte auftreten und zu geringer Ausbeute an verestertem Alkohol führen. Auch bereitet die Aufarbeitung der Reaktionsprodukte Schwierigkeiten, insbesondere die Trennung von Wasser und Kohlensäureester aus dem Reaktionsgemisch von dem homogenen Katalysator, dessen Ligand normalerweise eine organische Base ist. In der DE-A 2 743 690 wird als Lösung die Umsetzung des Alkohols mit Sauerstoff und Kohlenmonoxid in Gegenwart der einfachen Salze eines Metalls der Gruppe IB, IIB oder VIII des Periodensystems, dessen Kation an die geringstmögliche Anzahl anorganischer Anionen gebunden ist, angegeben. Die DE-A 3 045 767 beschreibt die Herstellung von Dimethylcarbonat durch Umsetzung mit denselben Metallverbindungen in Methanol unter Verwendung des wasserstoffhaltigen Synthesegases. Bei den letzten beiden genannten Druckschriften scheint nur bei erhöhtem Reaktionsdruck (20–30 bar) eine katalytische Reaktion mit relativ geringer Aktivität abzulaufen. Der «Turnover» von CuCl beträgt etwa 4–6 Katalysecyclen. Für eine technische Synthese müssten erhebliche Mengen des Kupfersalzes eingesetzt werden.

Bei den Verfahren gemäss DE-B 2 334 736, DE-A 2 431 330 und 2 520 708 werden Reaktionsdrucke von 40–100 bar und Temperaturen zwischen 140 und 190°C angewendet. Diese Verfahren führen zwar zu vergleichsweise guten bis sehr guten Umsätzen, bringen aber eine erhebliche Beanspruchung der verwendeten Reaktoren mit sich. Bei Verwendung von Stahlautoklaven muss z.B. mit beträchtlicher Korrosion gerechnet werden.

Die US-PS 3 114 762 lehrt eine Kombination der Metallsalze $PdCl_2/CuCl_2$ bei der Umsetzung von Kohlenmonoxid und Alkohol (jedoch ohne Sauerstoff) zu den Kohlensäureestern. Bei 1 bar Reaktionsdruck erfolgt eine ausschliesslich stöchiometrische Umsetzung. Die bisher bekannten Verfahren erzeugen Kohlensäureester bei 1 bar Reaktionsdruck – bezogen auf den eingesetzten Katalysator – ausschliesslich stöchiometrisch.

Aufgabe

Der Stand der Technik gibt Verfahren an, deren technische Realisierung erhebliche Schwierigkeiten bereitet. Als wenig aussichtsreich müssen von vorneherein Verfahren betrachtet werden, bei denen die Metallverbindungen in stöchiometrischen Mengen an der Umsetzung beteiligt sind. Weiter war die Anwendung hoher Drucke und Temperaturen zu vermeiden, u.a. weil dabei zu starke Korrosion auftritt. Ein technisch zu realisierendes Verfahren sollte demnach ein im echten Sinne katalytisches Verfahren sein, das unter möglichst milden Reaktionsbedingungen, insbesondere niedrigem Reaktionsdruck, zu guten Ausbeuten an Kohlensäureestern führt.

Lösung

Zur Lösung der vorliegenden Aufgabe wird das Verfahren gemäss Hauptanspruch vorgeschlagen. Hervorgehoben sei, dass hinsichtlich der Auswahl der Alkohole ROH keine Beschränkungen zu erkennen sind. Die Alkohole können beispielsweise einfach oder mehrfach ungesättigt sein. Es kann sich ferner um primäre oder sekundäre Alkohole sowie um Diole und Polyole handeln. Die Alkohole können weiter Substituenten tragen, z.B. auch funktionelle Gruppen, wie beispielsweise die Dialkylamingruppe. Die Eignung des Verfahrens zur Herstellung des Methylesters, Ethylesters, Propylesters, Isopropylesters, Butylesters, Benzylesters und Cyclohexylesters sei hervorgehoben.

In der Regel wird die Umsetzung ohne Lösungsmittel, d.h. in dem aus den Reaktionspartnern gebildeten Reaktionsgemisch durchgeführt, jedoch können auch geeignete, inerte Lösungsmittel verwendet werden.

Die Umsetzung wird bei einem Reaktionsdruck unterhalb 20 bar, bevorzugt unterhalb 15 bar, speziell im Bereich von 1 bis 10 bar, durchgeführt. Im allgemeinen werden Temperaturen im Bereich von 20 bis 300°C, vorzugsweise 40 bis 200°C angewendet. Das Verhältnis CO zu $O_2$ kann an sich in weiten Grenzen variiert werden; wegen des stöchiometrisch vorgezeichneten Verhältnisses werden Kohlenmonoxid und Sauerstoff

zweckmässig im (Druck-)Verhältnis 2:1 eingeführt.

Ausführung der Erfindung

Die Katalysatoren:

Die Katalysatoren der Formeln II und IIA weisen in der Bedeutung X=Halogen, vorzugsweise Chlorid, Bromid oder Jodid auf, gleiches gilt für die Katalysatoren der Formel II in der Bedeutung Y.

Die Bedeutungen von X und Y können sowohl gleich als auch verschieden sein. Besonders bevorzugt ist der Fall, wenn Y für Brom steht, ebenfalls bevorzugt ist der Fall, wenn X für Brom steht. Bei den Palladiumsalzen der Formel IIA steht M′ vorzugsweise für ein Alkalikation, wie $Li^+$, $Na^+$, $K^+$, $Rb^+$, $Cs^+$ oder ein Ammoniumkation. Besonders hervorgehoben seien als Verbindungen der Formel IIA diejenigen, worin M′ für Kalium steht. Genannt seien die Verbindungen $K_2PdCl_4$, $K_2PdBr_4$ und $K_2PdJ_4$. Für X und Y als Carboxylate kommen im Sinne der vorliegenden Erfindung die gleichen Bedeutungen in Frage, wie für den Rest

$$^{\ominus}O\overset{\overset{\displaystyle O}{\|}}{C}R_1$$

in Formel IV. Besonders genannt seien das Acetatanion, das Pivalatanion und das Stearatanion. Die der Formel IV zugrundeliegenden Carbonsäuren sind bevorzugt einbasische Carbonsäuren vom Typ der Ameisensäure bis zu C 30-Carbonsäuren.

Als Carbonsäuresalze der Formel IV kommen insbesondere diejenigen, worin $R_1$ für Wasserstoff oder einen gegebenenfalls verzweigten Alkylrest mit 1–6 Kohlenstoffatomen steht, speziell ein Methyl-, Ethyl-, n-Propyl-, iso-Propyl-, n-Butyl, iso-Butyl-, tert.-Butyl-, oder ein Cyclohexyl- oder Phenylrest in Frage, die gegebenenfalls partiell ungesättigt und/oder substituiert sein können. Die Substituenten umfassen unter den Reaktionsbedingungen inerte Gruppen. Sehr gut brauchbar sind jedoch auch längerkettige Reste $R_1$, z.B. solche mit 10 bis 30 C-Atomen, speziell mit 12, 16 und 17 C-Atomen. Als Kation M″ kommen die gleichen Kationen in Frage wie für M′, jedoch brauchen M′ und M″ nicht identisch zu sein.

Die Katalysatorkomponenten der Formel II, III und IV stehen vorteilhafterweise im molaren Verhältnis (0,1 bis 0,3) zu 1 zu (1 bis 3).

Im allgemeinen stehen die Katalysatoren insgesamt zu den Alkohol ROH im ungefähren molaren Verhältnis $1:10^3$, wobei Abweichungen nach oben und unten möglich sind.

Durchführung:

Die geringen erforderlichen Drucke gestatten es, die Reaktion ohne Anwendung der sonst gebräuchlichen teuren Druckapparaturen durchzuführen. Das erfindungsgemässe Verfahren kann z.B. in Glasautoklaven oder emaillierten Druckkesseln durchgeführt werden. Insgesamt tritt das Korrosionsproblem, das den stand der Technik so stark belastet, in den Hintergrund. In der Regel wird ein $CO/O_2$-Gasgemisch, vorzugsweise bei 1 bis 10 bar, in den die Katalysator-Kombination enthaltenden Alkohol der Formel I eingeleitet.

Vorteilhafterweise wird das Gasgemisch mittels einer Pumpe im Kreis durch die im Reaktor befindliche Reaktionsmischung geführt. Bei einer Reaktionstemperatur von 50°C kann als Richtwert für die Reaktionsdauer ca. 2–7 h, in vielen Fällen 2–3 h, angenommen werden. Alternativ kann auch dem Reaktionsansatz (vorzugsweise nach erfolgter Spülung mit einem Inertgas wie Stickstoff) das Kohlenmonoxid und der Sauerstoff aufgepresst werden. Nach einer gewissen Reaktionsdauer können erneut Mengen der beiden Gase aufgepresst werden, beispielsweise drei- bis viermal insgesamt. Die Ausbeutebestimmung kann in an sich bekannter Weise, beispielsweise qualitativ durch kombinierte Gaschromatographie/Massenspektrometrie und quantitativ durch Gaschromatographie vorgenommen werden.

Vorteilhafte Wirkungen

Das Verfahren gemäss der vorliegenden Erfindung gestattet die Synthese der Kohlensäureester mit geringem apparativem Aufwand und mit günstiger Energiebilanz. Insbesondere tritt das Problem der Korrosion in den Hintergrund. Die Reaktionsprodukte zeichnen sich durch sehr gute Ausbeuten und hohe Reinheit aus.

Der synergistische Effekt der Carbonsäuresalze der Formel IV, im Zusammenwirken mit den Katalysatoren der Formeln II bzw. IIA und III, konnte in keiner Weise vorausgesehen werden. Der synergistische Effekt kommt z.B. in den Beispielen 15–17, im Vergleich zu Beispiel 8, oder im Beispiel 18, im Vergleich zu Beispiel 19, zum Ausdruck.

Beispiele

Ausführung für die Beispiele 1–17

In einem heizbaren Reaktor mit Gaseinleitrohr wurden 50 ml Methanol als Lösungsmittel und 0,85 mmol Pd-Salz, 3,7 mmol Cu-Salz sowie 5,1 mmol Kaliumacetat als Katalysator vorgelegt. Danach wurde bei 50°C und 1 bar ein $CO:O_2=2:1$ Gasgemisch in die Reaktionsmischung eingeleitet. Das Gasgemisch wurde mit einer Pumpe durch die Reaktionsmischung im Kreis gefahren. Die Hauptreaktion war nach 2–3 h beendet.

Nach dem Abkühlen wurde Dimethylcarbonat (DMC) mit Hilfe der Gaschromatographie (GC) identifiziert und quantitativ bestimmt.

Ergebnisse siehe Tabelle 1.

Tabelle 1

Reaktionsbedingungen: Temperatur: 50° C, Druck: 1 bar $CO:O_2 = 2:1$

| Beispiel | Katalysator | g DMC | Gew.% DMC im Reaktor-austrag | Umsatz (%) bez. auf $CH_3OH$ | mmol DMC/ mmol Pd-Salz | mmol DMC/ mmol Cu-Salz |
|---|---|---|---|---|---|---|
| 1 | $K_2PdCl_4/CuCl/KOAc$ | 3,3 | 8,0 | 6,1 | 43 | 9,9 |
| 2 | $K_2PdCl_4/CuBr/KOAc$ | 4,7 | 11,4 | 8,7 | 61 | 14,1 |
| 3 | $K_2PdCl_4/CuJ/KOAc$ | 0,1 | 0,3 | 0,2 | 1 | 0,3 |
| 4 | $K_2PdCl_4/CuOAc/KOAc$ | 3,4 | 8,4 | 6,3 | 44 | 10,2 |
| 5 | $K_2PdCl_4/Cu(OAc)_2/KOAc$ | 3,0 | 7,4 | 5,6 | 39 | 9,0 |
| 6 | $K_2PdCl_4/CuCl_2/KOAc$ | 1,4 | 3,4 | 2,6 | 18 | 4,2 |
| 7 | $K_2PdBr_4/CuCl/KOAc$ | 3,3 | 8,0 | 6,1 | 43 | 9,9 |
| 8 | $K_2PdBr_4/CuBr/KOAc$ | 5,2 | 12,6 | 9,7 | 68 | 15,6 |
| 9 | $K_2PdBr_4/Cu(OAc)KOAc$ | 4,4 | 10,8 | 8,2 | 58 | 13,2 |
| 10 | $K_2PdJ_4/CuCl/KOAc$ | 2,1 | 5,0 | 3,8 | 27 | 6,3 |
| 11 | $K_2PdJ_4/CuBr/KOAc$ | 4,2 | 10,2 | 7,8 | 55 | 12,6 |
| 12 | $PdBr_2/CuBr/KOAc$ | 4,2 | 10,1 | 7,8 | 55 | 12,6 |
| 13 | $PdCl_2/CuCl/KOAc$ | 1,6 | 4,0 | 3,0 | 21 | 4,8 |
| 14 | $Pd(OAc)_2/CuCl/KOAc$ | 1,6 | 4,0 | 3,0 | 21 | 4,8 |
| 15 | $PdBr_2/CuBr$ | < 0,1 | < 0,1 | – | – | – |
| 16 | $PdBr_2$ | < 0,1 | < 0,1 | – | – | – |
| 17 | CuBr | < 0,1 | < 0,1 | – | – | – |

Ausführung für die Beispiele 18–25

Es wurde wie in den Beispielen 1–17 gearbeitet. Zu 0,85 mmol Pd-Salz und 3,7 mmol Cu-Salz in 50 ml Methanol wurden jeweils 5,1 mmol eines Alkalicarboxylates gegeben.

Ergebnis siehe Tabelle 2.

Tabelle 2

Reaktionsbedingungen: Temperatur: 50° C, Druck: 1 bar $CO:O_2 = 2:1$

| Beispiel | Katalysator | g DMC | Gew.% DMC im Reaktor-austrag | Umsatz (%) bez. auf $CH_3OH$ | mmol DMC/ mmol Pd-Salz | mmol DMC/ mmol Cu-Salz |
|---|---|---|---|---|---|---|
| 18 | $PdBr_2/CuBr$ | <0,1 | < 0,1 | – | – | – |
| 19 | $PdBr_2/CuBr/KOAc$ | 4,2 | 10,1 | 7,8 | 55 | 13 |
| 20 | $PdBr_2$/CuBr/K-pivalat | 2,7 | 6,7 | 5,1 | 35 | 8 |
| 21 | $PdBr_2$/CuBr/K-stearat | 2,5 | 6,0 | 4,7 | 33 | 7 |
| 22 | $Pd(OAc)_2/CuCl/LiOAc$ | 1,2 | 3,0 | 2,2 | 16 | 4 |
| 23 | $Pd(OAc)_2/CuCl/NaOAc$ | 1,1 | 2,6 | 2,1 | 14 | 3 |
| 24 | $Pd(OAc)_2/CuCl/KOAc$ | 2,1 | 5,1 | 3,9 | 27 | 6 |
| 25 | $Pd(OAc)_2/CuCl/RbOAc$ | 1,1 | 2,7 | 2,1 | 14 | 3 |

Ausführung für die Beispiele 26–30

In einem 2 l-Glasautoklaven wurden 150 ml Methanol und 2,55 mmol $K_2PdBr_4$, 11,1 mmol CuBr und 15,3 mmol Kaliumpivalat vorgelegt. Nach Spülung mit $N_2$ wurden bei Raumtemperatur 4 bar CO und 2 bar $O_2$ aufgepresst. Danach wurde auf 40–160°C erhitzt. Insgesamt wurden 3 mol CO und $O_2$ im angegebenen Verhältnis aufgepresst. Nach dem Abkühlen und Entspannen wurde der Reaktoraustrag jeweils quantitativ gaschromatographisch analysiert.

Ergebnisse siehe Tabelle 3.

Tabelle 3

Reaktionsbedingungen: Temperaturen zwischen 40 und 160° C; Druck: 6–10 bar, $CO:O_2 = 2:1$

| Beispiel | T (° C) | (min) | g DMC | Gew.% DMC im Reaktor-austrag | Umsatz (%) bez. auf $CH_3OH$ | mmol DMC/ mmol Pd-Salz | mmol DMC/ mmol Cu-Salz |
|---|---|---|---|---|---|---|---|
| 26 | 40 | 90 | 20,9 | 16,0 | 13,1 | 93 | 21 |
| 27 | 80 | 100 | 30,3 | 22,0 | 18,9 | 135 | 30 |
| 28 | 120 | 55 | 31,0 | 22,8 | 19,4 | 138 | 31 |
| 29 | 160 | 30 | 22,4 | 16,7 | 14,0 | 100 | 22 |
| 30 | *120 | 80 | 48,7 | 33,4 | 30,4 | 108 | 24 |

* doppelte Katalysatormenge.

Ausführung für die Beispiele 31 und 32

Bis 10 bar Reaktionsdruck erfolgten die Versuche im 1 l-Glasautoklaven und ab 10 bar im 0,35 l-Stahlautoklaven, jeweils mit Innentemperaturmessung und Magnetrührung.

Es wurden 50 ml Methanol als Lösungsmittel und 0,85 mmol $Pd(OAc)_2$, 3,7 mmol CuCl und 5,1 mmol K-pivalat als Katalysator vorgelegt. Danach wurde bei Raumtemperatur bis zu dem in der Tabelle 4 angegebenen Druck CO und $O_2$ im Verhältnis 2:1 aufgepresst. Anschliessend wurde auf 60°C Reaktionstemperatur erhitzt. Nach Versuchsende wurde der Autoklav bei Raumtemperatur entspannt und das erhaltene Reaktionsgemisch mit Hilfe der Gaschromatographie quantitativ bestimmt.

Ergebnisse siehe Tabelle 4.

Tabelle 4

| Beispiel | p (bar) | (g) | Dimethylcarbonat Gew.% im Reaktoraustrag | Umsatz (%) bez. auf $CH_3OH$ | (g) | Oxalsäure Gew.% im Reaktoraustrag |
|---|---|---|---|---|---|---|
| 31 | 3 | 3,9 | 9,9 | 7,3 | 0,0 | 0,0 |
| 32 | 6 | 6,6 | 15,8 | 12,4 | 0,0 | 0,0 |

Ausführung für die Beispiele 33–40

In einem heizbaren Reaktor mit Gaseinleitrohr wurden 50 ml Alkohol (s. Tab. 5) und 0,85 mmol $PdBr_2$, 3,7 mmol CuBr sowie 5,1 mmol KOAc als Katalysator vorgelegt. Danach wurde bei 50°C und 1 bar ein $CO:O_2 = 2:1$ Gasgemisch in die Reaktionsmischung eingeleitet. Das Gasgemisch wurde mit einer Pumpe 7 h durch die Reaktionsmischung im Kreis gefahren.

Nach dem Abkühlen wurden die Carbonate durch Gaschromatographie/Massenspektrometrie und mit GC quantitativ bestimmt.

Ergebnisse siehe Tabelle 5.

Tabelle 5

(Katalysator $PdBr_2$/CuBr/KOAc; 50 ml ROH; 7 h; Druck: 1 bar $CO:O_2 = 2:1$)

| Beispiel | Alkohol ROH | T (° C) | Kohlensäureester | | Umsatz (%) bez. auf ROH | mmol Carbonat/ mmol Pd-Salz | mmol Carbonat/ mmol Cu-Salz |
|---|---|---|---|---|---|---|---|
| | | | (g) | Gew.% im Reaktor-austrag | | | |
| 33 | $C_2H_5OH$ | 75 | 2,8 | 7,6 | 5,5 | 27,9 | 6,5 |
| 34 | $C_3H_7OH$ | 75 | 5,3 | 13,7 | 10,9 | 42,6 | 9,8 |
| 35 | i-$C_3H_7OH$ | 75 | 1,4 | 3,7 | 2,9 | 11,2 | 2,6 |
| 36 | n-$C_4H_9OH$ | 100 | 10,2 | 23,4 | 21,2 | 69,0 | 15,8 |
| 37 | 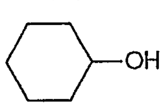 | 100 | 6,2 | 15,0 | 11,7 | 32,2 | 7,4 |

Tabelle 5 (Fortsetzung)

| Beispiel | Alkohol ROH | T (° C) | Kohlensäureester (g) | Kohlensäureester Gew.% im Reaktor-austrag | Umsatz (%) bez. auf ROH | mmol Carbonat/ mmol Pd-Salz | mmol Carbonat/ mmol Cu-Salz |
|---|---|---|---|---|---|---|---|
| 38 | 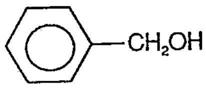 | 100 | 3,7 | 7,2 | 6,3 | 17,9 | 4,1 |
| 39 | $CH_3$-O-$CH_2$-$CH_2$-OH | 100 | 6,4 | 12,3 | 11,3 | 42,3 | 9,7 |
| 40 | HO-$CH_2$-$CH_2$-OH | 100 | 2,4* | 4,1 | 3,0 | 32,1 | 7,4 |

* 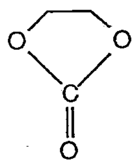 als Produkt.

Ausführung für die Beispiele 41–45

In einen 2 l-Glasautoklaven wurden 150 ml Alkohol (s. Tab. 6) und 2,55 mmol $K_2PdBr$, 11,1 mmol CuBr und 15,3 mmol KOAc vorgelegt. Nach Spülung mit $N_2$ wurden bei Raumtemperatur 4 bar CO und 2 bar $O_2$ aufgepresst. Danach wurde auf 100°C erhitzt. Insgesamt wurden dreimal CO und $O_2$ im angegebenen Verhältnis aufgepresst.

Nach dem Abkühlen und Entspannen wurde der Reaktoraustrag jeweils gaschromatographisch analysiert.

Ergebnis siehe Tabelle 6.

Tabelle 6
(Katalysator $K_2PdBr_4$/CuBr/KOAc; 150 ml ROH/100° C; Druck: 6–10 bar CO:$O_2$ = 2:1)

| Beispiel | Alkohol ROH | Zeit (h) | Kohlensäureester (g) | Kohlensäureester Gew.% im Reaktor-austrag | Umsatz (%) bez. auf ROH | mmol Carbonat/ mmol Pd-Salz | mmol Carbonat/- mmol Cu-Salz |
|---|---|---|---|---|---|---|---|
| 41 | $C_2H_5OH$ | 3 | 42 | 33,1 | 27,5 | 140 | 32 |
| 42 | i-$C_3H_7OH$ | 3,5 | 20 | 16,4 | 14,0 | 54 | 12 |
| 43 | n-$C_4H_9OH$ | 2,5 | 22 | 17,5 | 15,4 | 50 | 11 |
| 44 | 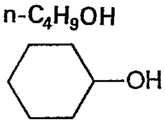 | 5 | 19 | 12,8 | 12,0 | 33 | 8 |
| 45 | 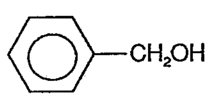 | 7 | 42 | 26,4 | 24,0 | 68 | 16 |

**Patentansprüche**

1. Verfahren zur Herstellung von Kohlensäureestern der allgemeinen Formel I

R O-C-O R (I)
    ||
    O

in der R einen gegebenenfalls ungesättigten, gegebenenfalls substituierten Alkyl-, Cycloalkyl-, oder Aralkylrest bedeutet oder beide Reste zusammen einen Ring bilden, durch Umsetzung eines Alkohols oder Diols ROH, wobei R die oben angegebene Bedeutung besitzt, mit Kohlenmonoxid und Sauerstoff in Gegenwart eines zum Teil aus Kupfer bestehenden Katalysators, dadurch gekennzeichnet, dass die Umsetzung in Gegenwart von Katalysatoren, bestehend aus einer Kobination von Palladiumsalzen der Formel II

$PdX_2$ (II)

worin X für ein Carboxylat- oder Halogenanion steht, mit Kupfersalzen der Formel III

$CuY_n$ (III)

worin Y für ein Carboxylat- oder Halogenanion und n (in Abhängigkeit von der Wertigkeit des Kupfers) für 1 oder 2 steht oder aus einer Kombination von Palladiumsalzen der Formel IIA

$M'_2PdX_4$ (IIA)

worin M′ für ein Metallkation steht und X die oben angegebene Bedeutung besitzt, mit der Verbindung der Formel III, worin n für 1 steht; und zusätzlich in Gegenwart eines oder mehrerer Carbonsäuresalze IV

$$M''\,O\overset{\overset{\displaystyle O}{\|}}{C}-R_1 \qquad (IV)$$

worin $R_1$ für Wasserstoff, einen gegebenenfalls verzweigten, gegebenenfalls ungesättigten und/oder cyclischen, gegebenenfalls substituierten Alkylrest mit 1 bis 30 Kohlenstoffatomen, einen gegebenenfalls substituierten Arylrest oder einen Aralkylrest mit 1 bis 30 Kohlenstoffatomen und M″ für ein Alkali-Kation steht und bei einem Reaktionsdruck unterhalb 20 bar durchgeführt wird.

2. Verfahren gemäss Anspruch 1, dadurch gekennzeichnet, dass die Umsetzung bei einem Reaktionsdruck unterhalb 15 bar, vorzugsweise im Bereich von 1 bis 10 bar, durchgeführt wird.

3. Verfahren gemäss den Ansprüchen 1 und 2, dadurch gekennzeichnet, dass die Umsetzung im Temperaturbereich von 20 bis 300°C durchgeführt wird.

4. Verfahren gemäss Anspruch 3, dadurch gekennzeichnet, dass die Umsetzung bei 40 bis 200°C durchgeführt wird.

5. Verfahren gemäss den Ansprüchen 1 bis 4, dadurch gekennzeichnet, dass das Reaktionsmedium im wesentlichen von Reaktionspartnern gebildet wird.

6. Verfahren gemäss den Ansprüchen 1 bis 5, dadurch gekennzeichnet, dass die Katalysatoren der Formel II, III und IV im molaren Verhältnis (0,1 bis 0,3):1:(1 bis 3) stehen.

7. Verfahren gemäss den Ansprüchen 1 bis 6, dadurch gekennzeichnet, dass die Gesamtheit der Katalysatoren zum Alkohol ROH im ungefähren molaren Verhältnis 1:$10^3$ steht.

8. Verfahren gemäss den Ansprüchen 1 bis 7, dadurch gekennzeichnet, dass ein Gemisch aus Kohlenmonoxid und Sauerstoff in die aus dem Alkohol ROH und den Kalaysatoren der Formeln II und III bzw. IIA und IV bestehnde Reaktionsmischung eingeleitet wird.

9. Verfahren gemäss den Ansprüchen 1 bis 8, dadurch gekennzeichnet, dass in den Katalysatoren der Formel IV $R_1$ für Wasserstoff oder einen gegebenenfalls verzweigten Alkylrest mit 1 bis 6 Kohlenstoffatomen, vorzugsweise Methyl-, Ethyl-, n-Propyl-, Isopropyl-, n-Butyl-, iso-Butyl, tert.-Butyl-, oder einen Cyclohexyl- oder Phenylrest steht.

10. Verfahren gemäss den Ansprüchen 1 bis 8, dadurch gekennzeichnet, dass in den Katalysatoren der Formel IV $R_1$ für einen längerkettigen Alkylrest mit 10 bis 30 Kohlenstoffatomen, vorzugsweise 12, 16 oder 17 Kohlenstoffatomen steht.

11. Verfahren gemäss den Ansprüchen 1 bis 10, dadurch gekennzeichnet, dass R für einen Alkylrest mit 1 bis 4 Kohlenstoffatomen, einen Benzyl- oder einen Cyclohexylrest steht.

12. Verfahren gemäss Anspruch 11, dadurch gekennzeichnet, dass R für Methyl steht.

13. Verfahren gemäss Anspruch 12, dadurch gekennzeichnet, dass R für einen Rest $-C_4H_9$ steht.

## Claims

1. Process for preparing carbonic acid esters of general formula I

$$R\,O-\underset{\underset{\displaystyle O}{\|}}{C}-O\,R \qquad (I)$$

wherein R represents an optionally unsaturated, optionally substituted, alkyl, cycloalkyl or aralkyl group or both groups together form a ring, by reacting an alcohol or diol ROH wherein R is as hereinbefore defined with carbon monoxide and oxygen in the presence of a catalyst consisting partly of copper, characterised in that the reaction is carried out in the presence of catalysts consisting of a combination of palladium salts of formula II

$PdX_2$ (II)

wherein X represents a carboxylate or halogen anion, with copper salts of formula III

$CuY_n$ (III)

wherein Y represents a carboxylate or halogen anion and n represents 1 or 2 (depending on the valency of the copper) or consisting of a combination of palladium salts of formula IIA

$M'_2PdX_4$ (IIA)

wherein M′ represents a metal cation and X is as hereinbefore defined, with the compound of formula III wherein n represents 1; and additionally in the presence of one or more carboxylic acid salts IV

$$M''\,O\overset{\overset{O}{\|}}{C}-R_1 \qquad (IV)$$

wherein $R_1$ represents hydrogen, an optionally branched, optionally unsaturated and/or cyclic, optionally substituted alkyl group with 1 to 30 carbon atoms, an optionally substituted aryl group or an aralkyl group with 1 to 30 carbon atoms and M″ represents an alkali cation, and the reaction is carried out under a reaction pressure of below 20 bar.

2. Process as claimed in claim 1, characterised in that the reaction is carried out under a reaction pressure of below 15 bar, preferably in the range from 1 to 10 bar.

3. Process as claimed in claims 1 and 2, characterised in that the reaction is carried out in the temperature range from 20 to 300°C.

4. Process as claimed in claim 3, characterised in that the reaction is carried out at 40 to 200°C.

5. Process as claimed in claims 1 to 4, characterised in that the reaction medium is formed essentially by reactants.

6. Process as claimed in claims 1 to 5, characterised in that the catalysts of formulae II, III and IV are in a molar ratio of (0.1 to 0.3):1:(1 to 3).

7. Process as claimed in claims 1 to 6, characterised in that all the catalysts are in an approximate molar ratio of 1:$10^3$ to the alcohol ROH.

8. Process as claimed in claims 1 to 7, characterised in that a mixture of carbon monoxide and oxygen is passed into the reaction mixture consisting of the alcohol ROH and the catalysts of formulae II and III or IIA and IV.

9. Process as claimed in claims 1 to 8, characterised in that in the catalysts of formula IV $R_1$ represents hydrogen or an optionally branched alkyl group with 1 to 6 carbon atoms, preferably methyl, ethyl, n-propyl, isopropyl, n-butyl, isobutyl, tert. butyl or a cyclohexyl or phenyl group.

10. Process as claimed in claims 1 to 8, characterised in that in the catalysts of formula IV $R_1$ represents a longer-chained alkyl group with 10 to 30 carbon atoms, preferably 12, 16 or 17 carbon atoms.

11. Process as claimed in claims 1 to 10, characterised in that R represents an alkyl group with 1 to 4 carbon atoms or a benzyl or cyclohexyl group.

12. Process as claimed in claim 11, characterised in that R represents methyl.

13. Process as claimed in claim 11, characterised in that R represents a group $-C_4H_9$.

**Revendications**

1. Procédé pour la préparation d'esters d'acide carbonique de formule générale I

$$RO-\underset{\underset{O}{\|}}{C}-OR \qquad (I)$$

dans laquelle R est un radical alkyle, cycloalkyle ou aralkyle éventuellement insaturé, éventuellement substitué ou les deux radicaux forment ensemble un noyau, par réaction d'un alcool ou d'un diol ROH (R ayant la signification donnée ci-dessus) avec l'oxyde de carbone et l'oxygène en présence d'un catalyseur composé en partie de cuivre, caractérisé en ce que la réaction est effectuée en présence de catalyseurs constitués d'une combinaison de sels de palladium de formule II

$$PdX_2 \qquad (II)$$

dans laquelle X est mis pour un anion carboxylate ou halogène, avec des sels de cuivre de formule III

$$CuY_n \qquad (III)$$

dans laquelle Y est mis pour un anion carboxylate ou halogène et n pour 1 ou 2 (selon la valence du cuivre), ou d'une combinaison de sels de palladium de formule IIA

$$M'_2PdX_4 \qquad (IIA)$$

dans laquelle M′ est mis pour un cation métallique et X a la signification donnée ci-dessus, avec le composé de formule III dans laquelle n est mis pour 1, et en plus en présence d'un ou de plusieurs sels d'acides carboxyliques IV

$$M''\,O\overset{\overset{O}{\|}}{C}-R_1 \qquad (IV)$$

dans laquelle $R_1$ est mis pour un atome d'hydrogène, un radical alkyle à 1–30 atomes de carbone éventuellement ramifié, éventuellement insaturé et/ou cyclique, éventuellement substitué, un radical aryle éventuellement substitué ou un radical aralkyle à 1–30 atomes de carbone et M″ pour un cation alcali, et en ce que la réaction est menée sous une pression de réaction inférieure à 20 bars.

2. Procédé selon la revendication 1, caractérisé en ce que la réaction est menée sous une pression de réaction inférieure à 15 bars, de préférence dans la gamme de 1 à 10 bars.

3. Procédé selon l'une quelconque des revendications 1 et 2, caractérisé en ce que la réaction est menée dans la gamme de température de 20 à 300°C.

4. Procédé selon la revendication 3, caractérisé en ce que la réaction est menée entre 40 et 200°C.

5. Procédé selon l'une quelconque des revendications 1 à 4, caractérisé en ce que le milieu réactionnel est essentiellement constitué par des substances participant à la réaction.

6. Procédé selon l'une quelconque des revendications 1 à 5, caractérisé en ce que les catalyseurs de formules II, III et IV sont dans le rapport molaire (0,1 à 0,3):1:(1 à 3).

7. Procédé selon l'une quelconque des revendications 1 à 6, caractérisé en ce que l'ensemble des catalyseurs est, à l'alcool ROH, dans le rapport molaire approximatif 1:$10^3$.

8. Procédé selon l'une quelconque des revendications 1 à 7, caractérisé en ce qu'un mélange d'oxyde de carbone et d'oxygène est envoyé dans le mélange réactionnel composé de l'alcool ROH et des catalyseurs de formules II et III ou IIA et IV.

9. Procédé selon l'une quelconque des revendications 1 à 8, caractérisé en ce que dans les catalyseurs de formule IV, $R_1$ est mis pour un atome d'hydrogène ou un radical à 1–6 atomes de carbone éventuellement ramifié, de préférence un radical méthyle, éthyle, n-propyle, isopropyle, n-butyle, isobutyle, butyle tertiaire ou un radical cyclohexyle ou phényle.

10. Procédé selon l'une quelconque des revendications 1 à 8, caractérisé en ce que, dans les catalyseurs de formule IV, $R_1$ est mis pour un radical à longue chaîne à 10–30 atomes de carbone, de préférence à 12, 16 ou 17 atomes de carbone.

11. Procédé selon l'une quelconque des revendications 1 à 10, caractérisé en ce que R est mis pour un radical alkyle à 1–4 atomes de carbone, un radical benzyle ou un radical cyclohexyle.

12. Procédé selon la revendication 11, caractérisé en ce que R est mis pour un radical méthyle.

13. Procédé selon la revendication 11, caractérisé en ce que R est mis pour un radical $-C_4H_9$.